# EUROPÄISCHE PATENTSCHRIFT

(11) **EP 0 689 531 B1**
(45) Veröffentlichungstag und Bekanntmachung des Hinweises auf die Patenterteilung: **29.07.1998**
(21) Anmeldenummer: 94900123.4
(22) Anmeldetag: 10.11.1993
(51) Int. Cl.: C07C 213/06, C07C 219/06, C07C 219/08, A61K 7/06

(54) **VERFAHREN ZUR HERSTELLUNG FESTER ESTERQUATS MIT VERBESSERTEM EMULGIERVERMÖGEN**
METHOD OF PRODUCING SOLID QUATERNARY ESTERS WITH IMPROVED EMULSIFYING POWER
PROCEDE POUR LA PREPARATION D'ESTERS QUATERNAIRES SOLIDES A POUVOIR EMULSIONNANT AMELIORE

(30) Priorität: 18.03.1993 DE 4308794; 20.10.1993 DE 4335782
(43) Veröffentlichungstag der Anmeldung: 03.01.1996
(73) Patentinhaber: Henkel Kommanditgesellschaft auf Aktien, 40191 Düsseldorf (DE)
(72) Erfinder: BEHLER, Ansgar, D-46240 Bottrop (DE); FABRY, Bernd, D-41352 Korschenbroich (DE); PI, Rafael, E-08400 Granollers (ES); BIGORRA LLOSAS, Joaquim, E-08203 Sabadell (ES); PRAT QUERALT, Ester, E-08728 Barcelona (ES)
(86) Internationale Anmeldenummer: EP9303150
(87) Internationale Veröffentlichungsnummer: WO9421592

(56) Entgegenhaltungen:
- EP-A- 0 008 839
- WO-A-91/01295
- DE-A- 4 138 630

## Beschreibung

Die Erfindung betrifft ein Verfahren zur Herstellung fester Esterquats mit verbessertem Emulgiervermögen, die man erhält, indem man Fettsäuretriethanolaminester in Gegenwart von ausgewählten nichtionischen Emulgatoren quaterniert.

### Stand der Technik

Schädigungen der Haarstruktur sind die Folge häufigen Bleichens, Dauerwellens, Färbens, starker UV-Belastung, Waschens der Haare mit entfettenden Tensiden sowie das Ergebnis einer normalen Alterung. Das Haar wird spröde und verliert seinen Glanz. Des weiteren findet beim Kämmen des Haares eine elektrostatische Aufladung statt, während die aufgerauhte Haaroberfläche Anlaß zu Verfilzungen und Verknotungen des Haares gibt und auf diese Weise das Kämmen erschwert. Haarpflegemittel mit einer kämmbarkeitsverbessernden Wirkung haben daher erhebliche Bedeutung auf dem Kosmetikmarkt erlangt. Derartige Mittel können beispielsweise in Form einer Spülung, eines Aerosol-Schaums oder auch in Form von Emulsionen (Creme-Rinses) nach der Haarwäsche im noch nassen Haar verteilt und entweder nach einigen Minuten Einwirkungszeit ausgespült oder auf dem Haar belassen werden.

Als Wirkstoffe zur Verbesserung der Haarstruktur haben sich kationische Tenside, insbesondere quaternäre Ammoniumverbindungen wie beispielsweise Distearyldimethylammoniumchlorid (DSDMAC) alleine oder in Kombination mit verschiedenen wachs- artigen Zusätzen, wie Kohlenwasserstoffen, Fettalkoholen oder Fettsäureestern bewährt [**Parf.Kosm. 56, 157 (1975)**]. Von Nachteil ist hierbei jedoch, daß die genannten Kationtenside eine unzureichende biologische Abbaubarkeit aufweisen und somit bei Eintragung in Oberflächengewässer im Laufe der Zeit die Funktionsfähigkeit aquatischer Lebensgemeinschaften beeinträchtigen können.

Aus der deutschen Patentanmeldung **DE-A1 3527974** sind darüber hinaus Ester des Betains mit Fettalkoholen oder Fettalkoholpolyglycolethern für den Einsatz in sauren Haarpflegemitteln bekannt. Die Betainester weisen zwar eine hohe ökotoxikologische Verträglichkeit auf, sind jedoch im Hinblick auf Kämmbarkeitsverbesserung, Antistatik, Griff und Ausspülverhalten unbefriedigend und zudem im sauren Bereich nicht hydrolysestabil. In der älteren deutschen Patentanmeldung **DE-A1 4138630** hat die Anmelderin bereits vorgeschlagen, als kationische Tenside quaternierte Fettsäuretriethanolaminester-Salze, sogenannte "Esterquats", in sauren Haarpflegemitteln einzusetzen. In der Praxis hat sich jedoch gezeigt, daß sowohl die Emulgierbarkeit dieser Produkte in Wasser, als auch ihre Lagerstabilität nicht immer voll zufriedenstellend ist. Aus der europäischen Patentanmeldung **EP-A1 0008839** ist ferner ein Verfahren zur Quaternierung von Stickstoffverbindungen bekannt, bei dem die Reaktion in Gegenwart von nichtionischen Tensiden wie beispielsweise Sorbitanestern, Monoglyceriden und Fettalkoholethoxylaten als Phasentransferkatalysatoren erfolgt.

Die Aufgabe der Erfindung hat somit darin bestanden, ein Verfahren zur Herstellung von Esterquats mit verbesserter Emulgierbarkeit in Wasser und höherer Lagerbeständigkeit zu entwickeln, die sich mit Vorteil in Haarpflegemitteln einsetzen lassen.

### Beschreibung der Erfindung

Gegenstand der Erfindung ist ein Verfahren zur Herstellung fester Esterquats mit verbessertem Emulgiervermögen, bei dem man Fettsäuretriethanolaminester der Formel **(I)** 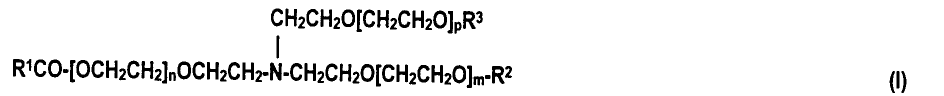 in der R¹CO für einen gesättigten und/oder ungesättigten Acylrest mit 6 bis 22 Kohlenstoffatomen, R² und R³ unabhängig voneinander für Wasserstoff oder R¹CO und n, m und p in Summe für 0 oder Zahlen von 1 bis 10 stehen, in Gegenwart nichtionischer Emulgatoren ausgewählt aus der Gruppe, die gebildet wird von
(a) Alkyl- und/oder Alkenyloligoglykosiden,
(b) Fettsäure-N-alkylpolyhydroxyalkylamiden,
(c) Partialglyceridpolyglycolethern und/oder
(d) Glycerin bzw. Oligoglycerin mit einem Eigenkondensationsgrad von 1,5 bis 10
in an sich bekannter Weise mit Alkylierungsmitteln quaterniert.

Überraschenderweise wurde gefunden, daß sich die Quaternierung von Fettsäuretriethanolaminestern auch in Gegenwart der genannten Emulgatoren durchführen läßt. Auf diese Weise werden lösemittelfreie, insbesondere alkoholfreie, feste Esterquats erhalten, die sich leicht in Wasser dispergieren lassen. Die Erfindung schließt die Erkenntnis ein, daß der nachträgliche Zusatz der genannten Emulgatoren zu nach konventionellen Verfahren hergestellten Esterquats das Emulgiervermögen nicht oder nur sehr geringfügig verbessert. Ein weiterer Vorteil besteht darin, daß die wäßrigen Lösungen der nach dem erfindungsgemäßen Verfahren erhältlichen Esterquats besonders lagerstabil sind, d.h., auch bei längerer Lagerung homogen bleiben und eine konstante Viskosität aufweisen. Nach Auflösen der erfindungsgemäßen Produkte in Wasser und pH-Werteinstellung werden beispielsweise unmittelbar Haarpflegemittel mit ausgezeichneten anwendungstechnischen Eigenschaften erhalten.

### Esterquats und Fettsäuretriethanolaminester

Esterquats stellen eine bekannte Gruppe kationischer Tenside dar, die üblicherweise durch Veresterung von Triethanolamin bzw. Triethanolaminpolyglycolethern mit Fettsäuren und nachfolgende Quaternierung in organischen Lösungsmitteln erhalten werden. Herstellung und Eigenschaften der Esterquats sind beispielsweise in der **WO 91/01295** (Henkel) sowie den Übersichtsartikeln von O.Ponsati in **C.R. CED-Kongress, Barcelona, 167 (1992)** und R.Puchta in **C.R. CED-Kongress, Sitges, 59 (1993)** beschrieben.

Im Sinne des erfindungsgemäßen Verfahrens kommen als Ausgangsstoffe für die Herstellung der Esterquats Fettsäuretri- ethanolaminester der Formel **(I)** in Betracht, die vorzugsweise technische Mono-/Di-/Triester-Gemische darstellen, bei denen der Veresterungsgrad im Bereich von 1,2 bis 2,5, insbesondere 1,5 bis 1,9 liegt. Besonders bevorzugt sind Ester, die sich von technischen C_{12/18}- bzw. C_{16/18} -Fettsäuren, wie beispielsweise Palmfettsäure, Kokosfettsäure oder Talgfettsäure ableiten und eine Iodzahl im Bereich zwischen 0 und 40 aufweisen können.

### Emulgatoren

(a) Als Emulgatoren kommen beispielsweise **Alkyl- und Alkenyloligoglykoside** der Formel **(I)** in Betracht, 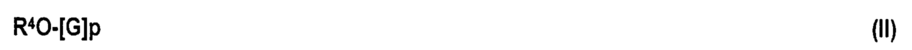 in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Hierbei handelt es sich um bekannte Stoffe, die nach den einschlägigen Verfahren der präparativen organischen Chemie erhalten werden können. Stellvertretend für das umfangreiche Schrifttum sei hier auf die Schriften **EP-A1 0301298** und **WO 90/3977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁴ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
(b) Als weitere Gruppe von Emulgatoren kommen **Fettsäure-N-alkylpolyhydroxyalkylamide** der Formel **(III)** in Frage, 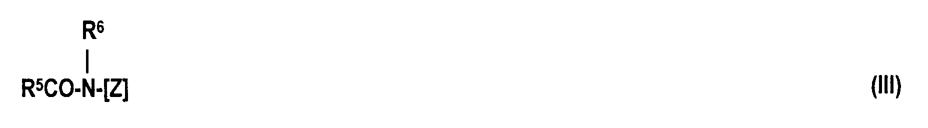 in der R⁵CO für einen aliphatischen Acylrest mit 6 bis 22 Kohlenstoffatomen, R⁶ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens.Surf.Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(IV)** wiedergegeben werden: 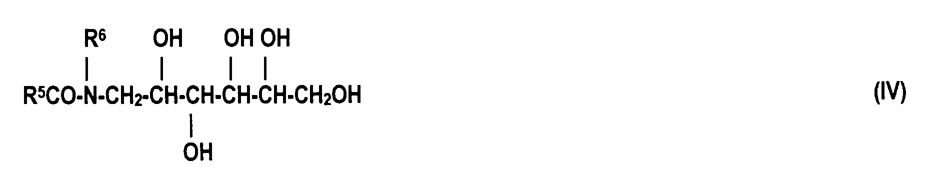 Vorzugsweise werden als Fettsäure-N-alkylpolyhydroxyalkylamide Glucamide der Formel **(IV)** eingesetzt, in der R⁶ für Wasserstoff oder eine Amingruppe steht und R⁵CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(IV)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.
(c) Als Emulgatoren können ferner auch **Partialglyceridpolyglycolether** eingesetzt werden. Hierbei handelt es sich beispielsweise um Anlagerungsprodukte von durchschnittlich 1 bis 10, vorzugsweise 2 bis 5 Mol Ethylenoxid an Fettsäuremono- bzw. -diglyceride. Typische Beispiele sind Ethylenoxidaddukte an technische C_{12/14}- bzw. C_{8/18}-Kokostettsäuremono- bzw. -diglyceride.
(d) Ebenfalls als Emulgatoren sind Polyolverbindungen geeignet, bei denen es sich um Glycerin oder Oligoglycerine mit einem Eigenkondensationsgrad von durchschnittlich 1,5 bis 10 handelt.

Wie zuvor schon erläutert, besteht der Kern der Erfindung darin, ein leicht emulgierbares Esterquat-Compound zu erzeugen, dem ein Emulgator schon während der Herstellung zugesetzt wird. Abgesehen von den bereits zuvor geschilderten anwendungstechnischen Vorteilen, wird auf diese Weise elegant die ansonsten erforderliche Mitverwendung eines organischen Lösungsmittels in der Quaternierungsstufe umgangen. Üblicherweise können die erfindungsgemäßen Esterquats die Emulgatoren in solchen Mengen enthalten, daß ihr Gewichtsanteil 5 bis 70, vorzugsweise 10 bis 30 Gew.-% - bezogen auf das Endprodukt - beträgt. Für die Einstellung eines gewünschten Emulgatorgehaltes im Endprodukt ist es erforderlich, die berechnete Menge Emulgator dem Ester vor der Quaternierung zuzusetzen. Die erforderlichen Verhältnisse zu berechnen, bleibt dem Fachmann überlassen, der hierzu nicht erfinderisch tätig werden muß.

### Alkylierung

Die Alkylierung der Fettsäuretriethanolaminester kann in an sich bekannter Weise durchgeführt werden. Hierzu wird der Ester vorgelegt und mit dem Alkylierungsmittel - das man üblicherweise in äquimolaren Mengen oder leichtem Unterschuß einsetzt - bei erhöhten Temperaturen gerührt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, Dialkylsulfate und Ethylenoxid - letzteres in Gegenwart von Dialkylphosphaten - in Form ihrer Chloride oder Methylsulfat-Salze sowie Esterquat-Salze, die mit 1 bis 5 Mol Ethylenoxid quaterniert worden sind.

### Gewerbliche Anwendbarkeit

Die nach dem erfindungsgemäßen Verfahren erhältlichen Esterquats sind leicht in Wasser emulgierbar. Die Emulsionen sind homogen und lagerbeständig und weisen ausgezeichnete anwendungstechnische Eigenschaften im Hinblick auf die Verbesserung der Kämmbarkeit und die Verminderung der elektrostatischen Aufladung von Haaren auf. Emulsionen, die die nach dem erfindungsgemäßen Verfahren erhältlichen Esterquats enthalten, weisen in der Regel 5 bis 70 Gew.-% Esterquats und 30 bis 95 Gew.-% Emulgatoren auf, wobei letztere ausgewählt sind aus der Gruppe, die von Alkyl- und/oder Alkenyloligoglykosiden, Fettsäure-N-alkylpolyhydroxyalkylamiden, Partialglyceridpolyglycolethern und/oder Glycerin oder Oligoglycerinen gebildet wird.

In kosmetischen Mitteln, insbesondere Haarpflegemitteln, können die nach dem erfindungsgemäßen Verfahren erhältlichen Esterquats in Mengen von 70 bis 100, vorzugsweise 80 bis 90 Gew.-% - bezogen auf den Feststoffgehalt der Mittel - enthalten sein. Unter Haarpflegemittel sind in diesem Zusammenhang beispielsweise Haarshampoos, Haarspülungen, Haarfestiger, Fönfestiger und dergleichen zu verstehen; vorzugsweise weisen die Mittel einen pH-Wert im Bereich von 3 bis 5, vorzugsweise 3 bis 4 auf.

### Herstellungsbeispiel

In einem 1-l-Dreihalskolben mit Rührer, Innenthermometer und Destillationsaufsatz wurden 324 g (1,2 mol) teilgehärtete C_{16/18}-Talgfettsäure (Iodzahl = 40), 149 g (1 mol) Triethanolamin und 1,4 g 50 Gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Talgfettsäuretriethanolaminester abgekühlt, der Reaktionsansatz entspannt und unter ständigem Rühren innerhalb von 15 min 1 Liter Luft durchgeleitet. Anschließend wurde in einem 500-ml-Dreihalskolben mit Rührer, Tropftrichter und Rückflußkühler eine Mischung von 70 g (0,155 mol) des Esters und 7 bis 30 g Emulgator vorgelegt und unter Rühren auf 45°C erhitzt (Gewichtsverhältnis Esterquat : Emulgator = 90 : 10 bis 70 : 30). Innerhalb von 2 h wurden 18,9 g (0,15 mol) Dimethylsulfat zugetropft. Nach Beendigung der Zugabe wurde die Mischung weitere 2 h bei 60°C gerührt und nichtumgesetztes DMS durch Zusatz von 0,4 g (0,005 mol) Glycin zerstört. Die wasserfreien Esterquat/Emulgator-Gemische wurden als hellfarbige, wachsartige Massen erhalten, die gegebenenfalls anschließend mechanisch geschuppt wurden.

### Anwendungstechnische Beispiele

Jeweils 7 g der wasserfreien, festen Esterquats aus wurden in 93 g Wasser gelöst und der pH-Wert der Mischungen auf 3,3 eingestellt. Die Emulsionsbildung erfolgte unter schwachem Rühren bei Raumtemperatur. In allen Fällen wurden homogene, kosmetisch elegante Emulsionen erhalten. Die Viskosität der Emulsionen wurde nach 1, 2 und 15 d Lagerung bestimmt (Brookfield RVT, Spindel 2, 25°C, 20 Upm). Die Ergebnisse sind in Tabelle 1 zusammengefaßt:

**Tabelle 1**

| **Viskositätsmessungen** | | | | | |
|---|---|---|---|---|---|
| **Bsp.** | **Emulgator** | **EQ:EM** | **Viskosität [mPas]** | | |
| | | | **1 d** | **2 d** | **15 d** |
| 1 | C_{8/16}-Alkyloligoglucosid | 70:30 | 25 | 25 | 25 |
| 2 | C_{12/14}-Kokosfettsäure-N-methylglucamid | 70:30 | 30 | 30 | 30 |
| 3 | Cetiol HE | 90:10 | 25 | 25 | 25 |
| 4 | Glycerin | 90:10 | 275 | 275 | 275 |
| 5 | Diglycerin | 80:20 | 180 | 180 | 180 |
| Legende: EQ:EM = Gewichtsverhältnis Esterquat : Emulgator | | | | | |

### Vergleichsversuche

Zum Vergleich wurde ein handelsübliches Esterquat (Dehyquart® AU 36, 90 Gew.-%ig in Isopropylalkohol, Fa.Pulcra S.A., Barcelona/ES) zunächst vom Lösungsmittel befreit, anschließend - also nachträglich - mit den genannten Emulgatoren vermischt. In allen Vergleichsversuchen zeigte sich, daß zur Emulsionsbildung eine deutlich höhere Scherleistung erforderlich war. Im Hinblick auf die Viskosität der Vergleichsemulsionen wurden zwar ähnliche Anfangswerte erreicht, jedoch schon nach kurzer Lagerung eine rasche Viskositätsabnahme beobachtet.

## Patentansprüche

1. Verfahren zur Herstellung fester Esterquats mit verbessertem Emulgiervermögen, bei dem man Fettsäuretriethanolaminester der Formel **(I)** 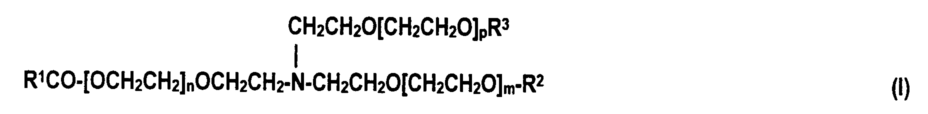 in der R¹CO für einen gesättigten und/oder ungesättigten Acylrest mit 6 bis 22 Kohlenstoffatomen, R² und R³ unabhängig voneinander für Wasserstoff oder R¹CO und n, m und p in Summe für 0 oder Zahlen von 1 bis 10 stehen, in Gegenwart nichtionischer Emul-gatoren ausgewählt aus der Gruppe, die gebildet wird von
(a) Alkyl- und/oder Alkenyloligoglykosiden,
(b) Fettsäure-N-alkylpolyhydroxyalkylamiden,
(c) Partialglyceridpolyglycolethern und/oder
(d) Glycerin bzw. Oligoglycerin mit einem Eigenkondensationsgrad von 1,5 bis 10
in an sich bekannter Weise mit Alkylierungsmitteln quaterniert.

2. Verfahren nach Anspruch 1, **dadurch gekennzeichnet**, daß man Alkyl- und/oder Alkenyloligoglykoside der Formel **(II)** einsetzt, 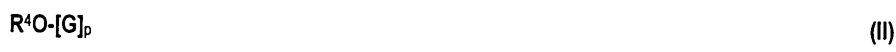 in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verfahren nach Anspruch 1, **dadurch gekennzeichnet**, daß man Fettsäure-N-alkylpolyhydroxyalkylamide der Formel **(III)** einsetzt, 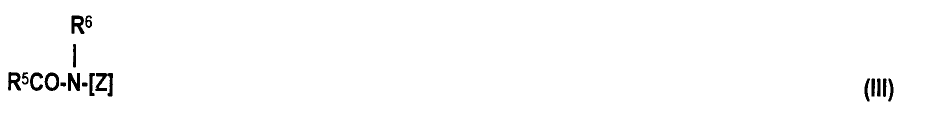 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Verfahren nach Anspruch 1, **dadurch gekennzeichnet**, daß man als Partialglyceridpolyglycolether Anlagerungsprodukte von durchschnittlich 1 bis 10 Mol Ethylenoxid an Fettsäuremono- bzw. -diglyceride einsetzt.

5. Verwendung von Esterquats erhältlich nach dem Verfahren nach Anspruch 1 zur Herstellung von kosmetischen Mitteln.

## Claims

1. A process for the production of solid esterquats having improved emulsifiability, in which fatty acid triethanolamine esters corresponding to formula (I): 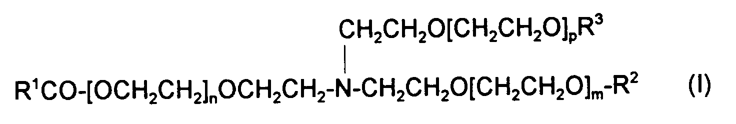 in which R¹CO is a saturated and/or unsaturated C₆₋₂₂ acyl radical, R² and R³ independently of one another represent hydrogen or have the same meaning as R¹CO and n, m and p together have a value of 0 or a value of 1 to 10, are quaternized with alkylating agents in known manner in the presence of nonionic emulsifiers selected from the group consisting of
(a) alkyl and/or alkenyl oligoglycosides,
(b) fatty acid N-alkyl polyhydroxyalkyl amides,
(c) partial glyceride polyglycol ethers and/or
(d) glycerol or oligoglycerol with a degree of self-condensation of 1.5 to 10.

2. A process as claimed in claim 1, characterized in that alkyl and/or alkenyl oligoglycosides corresponding to formula (II):
R⁴O-[G]ₚ (II)
in which R⁴ is an alkyl and/or alkenyl radical containing 4 to 22 carbon atoms, G is a sugar unit containing 5 or 6 carbon atoms and p is a number of 1 to 10, are used.

3. A process as claimed in claim 1, characterized in that fatty acid N-alkyl polyhydroxyalkyl amides corresponding to formula (III): 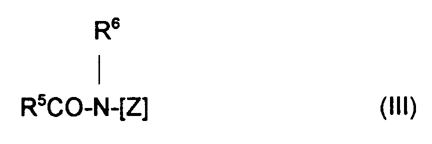 in which R⁵CO is an aliphatic acyl radical containing 6 to 22 carbon atoms, R⁶ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups, are used.

4. A process as claimed in claim 1, characterized in that adducts of on average 1 to 10 moles of ethylene oxide with fatty acid mono- or diglycerides are used as the partial glyceride polyglycol ethers.

5. The use of esterquats obtainable by the process claimed in claim 1 for the production of cosmetic formulations.

## Revendications

1. Procédé de fabrication d'esterquats solides ayant un pouvoir d'émulsification amélioré, dans lequel on quaternise de façon connue avec des agents d'alkylation des esters de triéthanolamine d'acide gras de formule **(I)** 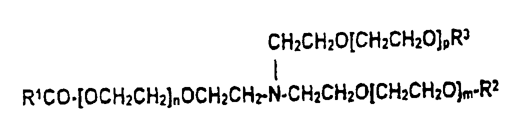 dans laquelle R¹CO représente un radical acyle saturé et/ou insaturé ayant de 6 à 22 atomes de carbone, R² et R³ représentent indépendamment l'un de l'autre un hydrogène ou R¹CO et la somme de n, m et p vaut 0 ou des nombres allant de 1 à 10, en présence d'émulsifiants non ioniques choisis dans le groupe formé par
(a) les alkyl- et/ou alcényl- oligoglycosides,
(b) les N-alkylpolyhydroxyalkylamides d'acides gras,
(c) les polyglycoléthers partiels de glycérides, et/ou
(d) le glycérol ou selon les cas l'oligoglycérol ayant un degré de condensation propre de 1,5 à 10.

2. Procédé selon la revendication 1,
caractérisé en ce qu'
on utilise des alkyl- et/ou alcényloligoglycosides de formule **(II)** 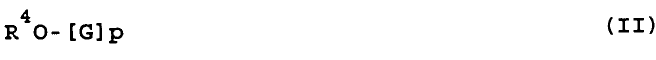 dans laquelle R⁴ représente un radical alkyle et/ou alcényle ayant de 4 à 22 atomes de carbone, G représente un radical saccharide ayant 5 ou 6 atomes de carbone et p représente des nombres allant de 1 à 10.

3. Procédé selon la revendication 1,
caractérisé en ce qu'
on utilise des N-alkylpolyhydroxyalkylamides d'acides gras de formule **(III)**, 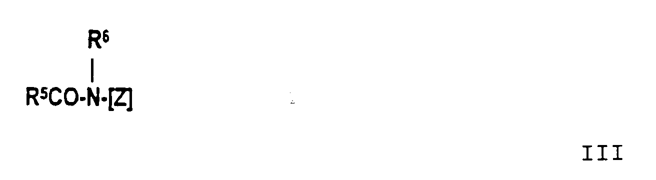 dans laquelle R⁵CO représente un radical acyle aliphatique avec C₆ à C₂₂ atomes de carbone, R⁶ représente un hydrogène, un radical alkyle ou hydroxyalkyle avec C₁ à C₄ atomes de carbone et [Z] représente un radical polyhydroxyalkyle linéaire ou ramifié en C₃ à C₁₂ atomes de carbone et avec de 3 à 10 groupes hydroxyle.

4. Procédé selon la revendication 1,
caractérisé en ce qu'
on utilise comme polyglycoléther partiel de glycéride des produits de fixation d'en moyenne 1 à 10 oxydes d'éthylène sur des mono- ou selon les cas diglycérides d'acides gras.

5. Utilisation d'esterquats que l'on peut obtenir selon le procédé de la revendication 1, pour la fabrication d'agents cosmétiques.
